(19) Europäisches Patentamt
European Patent Office
Office européen des brevets

(11) Veröffentlichungsnummer: **0 340 710**
**A2**

(12) # EUROPÄISCHE PATENTANMELDUNG

(21) Anmeldenummer: **89107886.7**

(22) Anmeldetag: **29.04.89**

(51) Int. Cl.[4]: **C07C 33/042 , C07C 29/42 , C07C 43/178 , C07C 69/145 , C07D 309/06 , C07F 7/08**

(30) Priorität: **04.05.88 DE 3815043**

(43) Veröffentlichungstag der Anmeldung:
**08.11.89 Patentblatt 89/45**

(84) Benannte Vertragsstaaten:
**CH DE ES FR IT LI NL**

(71) Anmelder: **BASF Aktiengesellschaft**
**Carl-Bosch-Strasse 38**
**D-6700 Ludwigshafen(DE)**

(72) Erfinder: **Huellmann, Michael, Dr.**
**Siegfriedstrasse 41**
**D-6148 Heppenheim(DE)**
Erfinder: **Becker, Rainer, Dr.**
**Im Haseneck 22**
**D-6702 Bad Duerkheim(DE)**
Erfinder: **Lauterbach, Gerald, Dr.**
**Breite Strasse 63**
**D-6700 Ludwigshafen(DE)**
Erfinder: **Buschmann, Ernst, Dr.**
**Georg-Ludwig-Krebs-Strasse 10**
**D-6700 Ludwigshafen(DE)**
Erfinder: **Eckhardt, Heinz, Dr.**
**Ruedigerstrasse 7**
**D-6700 Ludwigshafen(DE)**
Erfinder: **Himmele, Walter, Dr.**
**Eichenweg 14**
**D-6909 Walldorf(DE)**
Erfinder: **Brueckner, Christiane, Dr.**
**Bad-Aussee-Strasse 55**
**D-6700 Ludwigshafen(DE)**

(54) **3,9-Dihydroxynonin und dessen an der 9-OH-Funktion geschützte Derivate.**

(57) Die vorliegende Erfindung betrifft 3,9-Dihydroxynonin und dessen 9-0H-geschützten Derivate der allgemeinen Formel I

$$HC{\equiv}C-\underset{OH}{CH}-(CH_2)_6-OR \quad I,$$

in der R für Wasserstoff oder eine an sich übliche Alkoholschutzgruppe steht, Verfahren zur Herstellung der Verbindungen I und ihre Verwendung als Zwischenprodukte zur Synthese von E-7, Z-9-Dodecadienylacetat, dem Pheromon des bekreuzten Traubenwicklers.

EP 0 340 710 A2

## 3,9-Dihydroxynonin und dessen an der 9-0H-Funktion geschützte Derivate

Die vorliegende Erfindung betrifft 3,9-Dihydroxynonin und dessen 9-0H-geschützten Derivate der allgemeinen Formel I

$HC\equiv C-\underset{OH}{CH}-(CH_2)_6-OR$ I,

in der R für Wasserstoff oder eine an sich übliche basenstabile Alkoholschutzgruppe steht.

Die erfindungsgemäßen Verbindungen I dienen als Zwischenprodukte zur Synthese von E-7, Z-9-Dodecadienylacetat, dem Pheromon des bekreuzten Traubenwicklers, Lobesia Botrana. Dieses Pheromon wurde 1973 erstmals beschrieben, z.B. in US-A-3 845 108.

Das dort angegebene Herstellverfahren weist 10 meist aufwendige Verfahrensschritte auf und ist somit sehr umständlich. Zur Synthese größerer Mengen, wie sie zur großflächigen Verwendung von Pheromonwirkstoffen zur Insektenkontrolle mittels der Konfusionsmethode erforderlich sind, ist es nicht geeignet.

Der vorliegenden Erfindung liegt daher die Aufgabe zugrunde, ein wenig aufwendiges, technisch einfach durchführbares Herstellverfahren für den Pheromonwirkstoff zur Verfügung zu stellen.

Diese Aufgabe wurde durch Bereitstellung des eingangs beschriebenen neuen 3,9-Dihydroxynonins bzw. dessen 9-0H-geschützten Derivaten (Formel I) gelöst.

Als Schutzgruppen R eignen sich basenstabile Hydroxyschutzgruppen, z.B. $C_4$-$C_{20}$-, insbesondere $C_4$-$C_{12}$-tert.-Alkylgruppen, die in 1-Position ein tertiäres Kohlenstoffatom tragen, wie tert.-Butyl, 1,1-Dimethylprop-1-yl, 1,1-Dimethylbut-1-yl, 1,1,2-Trimethylprop-1-yl, 1,1-Dimethylpent-1-yl, 1,1,2-Trimethylbut-1-yl, 1,1,3-Trimethyl-but-1-yl, 1-Ethyl-1-methyl-but-1-yl, 1,1-Dimethylhex-1-yl und 1,1-Dimethyl-2-ethylbut-1-yl; $C_3$-$C_{20}$-Trialkylsilylgruppen, bevorzugt $C_3$-$C_8$-Trialklylsilylgruppen, wie Trimethylsilyl, Triethylsilyl, Tri-n-propylsilyl, Tri-isopropylsilyl, Tri-n-butylsilyl, Dimethylethylsilyl, Diethylmethylsilyl, Dimethyl-n-propylsilyl, Dimethyl-iso-propylsilyl, Dimethyl-n-butylsilyl und Dimethyl-tert.-butylsilyl; Benzyl; Benzoyl; $C_2$-$C_{10}$-Acyle, z.B. Alkanoyle wie Acetyl, Propionyl und Butyryl; Benzoyl, acyclische acetalische Gruppen, z.B. $C_2$-$C_{20}$-Alkoxymethoxy, bevorzugt $C_2$-$C_9$-Alkoxymethoxy wie Methoxymethoxy, Ethoxymethoxy, n-Propoxymethoxy, iso-Propoxymethoxy, n-Butoxymethoxy, iso-Butoxymethoxy, sec.-Butoxymethoxy, tert.-Butoxymethoxy, n-Hexoxymethoxy und n-Octoxymethoxy; $C_3$-$C_{20}$-1-Alkoxyethoxy, bevorzugt $C_3$-$C_{10}$-1-Alkoxy-ethoxy wie 1-Methoxy-ethoxy, 1-Ethoxy-ethoxy, 1-n-Propoxy-ethoxy, 1-iso-Propoxy-ethoxy, 1-n-Butoxy-ethoxy, 1-iso-Butoxy-ethoxy, 1-sec.-Butoxy-ethoxy, 1-tert.-Butoxy-ethoxy, 1-n-Hex-oxy-ethoxy und 1-n-Octoxyethoxy; cyclische acetalische Gruppen, insbesondere mit 5 oder 6 Ringgliedern wie 2-Furanyl, 2-Tetrahydrofuranyl, 2-Pyranyl, 2-Tetrahydropyranyl, 1,3-Dioxan-2-yl, 1,3-Dioxan-4-yl und 1,4-Dioxan-2-yl.

Die Herstellung der erfindungsgemäßen Verbindungen I erfolgt durch Umsetzung eines Aldehyds der Formel II,

$O=CH-(CH_2)_6-OR$ II,

in der R eine der oben genannten basenstabilen Alkoholschutzgruppen darstellt, mit Acetylen in einem aprotischen organischen Lösungsmittel in Gegenwart einer Base und gegebenenfalls anschließende Abspaltung der Schutzgruppe.

Hydroxyheptanale bzw. deren geschützte Derivate II sind bekannt oder lassen sich nach an sich bekannten Methoden, z.B. durch Hydroformylierung von geschützten $\omega$-Hex-1-enolen wie 6-tert.-Butoxyhex-1-en oder 6-Trimethylsilyloxy-hex-1-en in Gegenwart von Rhodium-Triphenylphosphinkomplexen als Katalysator, wobei vorteilhaft ein Überschuß an Triphenylphosphin verwendet wird, herstellen. Bevorzugte Bedingungen sind Temperaturen von 0 - 150 °C, insbesondere 20 - 60 °C; ein Synthesegasdruck von 50 - 200 bar und als Lösungsmittel inerte organische Lösungsmittel wie Kohlenwasserstoffe oder Ether, z.B. Tetrahydrofuran.

Pro Mol II können 1 bis 10 mol Acetylen verwendet werden.

Als Lösungsmittel können beispielsweise Kohlenwasserstoffe wie Benzol, Toluol, Xylol, Petrolether, Pentan oder Ether wie Diethylether, Methyl-tert.-butylether oder Tetrahydrofuran verwendet werden. Die Lösungsmittelmenge liegt im allgemeinen bei 1 bis 2 l pro Mol II.

Die Umsetzung erfolgt nach folgendem Reaktionsschema:

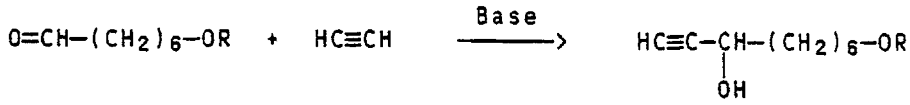


II I

R = OH-Schutzgruppe

Zur Herstellung des 3,9-Dihydroxynonins kann man die Alkoholschutzgruppen in an sich bekannter Weise (s. z.B. T.W. Greene, Protective Groups in Organic Synthesis, John Wiley, N.Y. 1981) abspalten.

Als Basen kommen Verbindungen in Betracht, die Acetylen zu deprotonieren vermögen wie beispielsweise Grignard-Verbindungen, z.B. Alkyl- oder Alkenylmagnesiumhalogenide wie Methylmagnesiumbromid, Methylmagnesiumiodid, Vinylmagnesiumbromid, Ethylmagnesiumchlorid oder Alkalimetallamide wie $LiNH_2$ oder $NaNH_2$. Pro Mol Acetylen können in der Regel 0,5 bis 1 mol, insbesondere 0,5 mol Base verwendet werden. Die Reaktionstemperaturen liegen im allgemeinen bei 0 bis 50 °C, insbesondere 0 bis 20 °C.

Die Aufarbeitung des Reaktionsgemisches erfolgt in an sich bekannter Weise, z.B. wäßrig.

Das Reaktionsprodukt kann z.B. destillativ oder chromatographisch gereinigt werden oder ohne Reinigung für Folgestufen eingesetzt werden. Will man die 9-OH-Funktion aus dem geschützten Derivat I freisetzen, so verfährt man wie z.B. in T.W. Greene, Protective Groups in Organic Synthesis, John Wiley, N.Y. 1981 allgemein beschrieben. In der Regel werden aber die geschützten Derivate weiterverwendet.

Die erfindungsgemäßen Verbindungen I können durch Meyer-Schuster-Umlagerung (Houben-Weyl, Methoden der Organischen Chemie, Bd. VII/2, S. 907-927, 1973) in die bekannten, ggf. OH-geschützten Nonenale III gemäß folgender Reaktionsgleichung

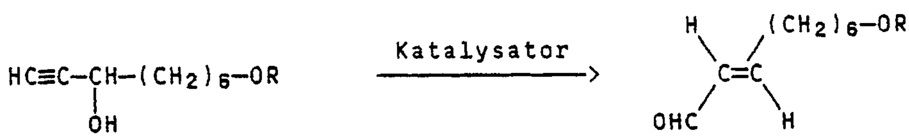


I III

R = H oder OH-Schutzgruppe

überführt werden. Die Verwendung dieser Nonenale III zur Synthese des gesuchten Pheromonwirkstoffes ist problemlos und beschrieben in GB-A-2 098 609 bzw. Liebigs Ann. Chem. 1705 - 1720 (1981).

Die nachfolgenden Beispiele veranschaulichen das Verfahren:

A) Allgemeine Vorschrift zur Darstellung der Acetylenalkohole Ia - Ie

In 1 l einer 1,5 molaren Vinylgrignard-Lösung werden unter Eiskühlung 3 mol Acetylen innerhalb von 2 Stunden eingeleitet. Anschließend tropft man 1 mol Aldehyd zu und rührt dann bei Raumtemperatur ca. 1 bis 2 Stunden weiter.

Zur Aufarbeitung werden 150 ml $H_2O$ zugetropft und das entstandene Magnesiumsalz abgesaugt. Das Filtrat wird am Rotationsverdampfer eingeengt und der Rückstand mit Methyl-tert.-butylether versetzt. Die etherische Lösung wird mit NaCl-Lösung gewaschen anschließend eingeengt und destilliert.

Gegebenenfalls kann auch das Rohprodukt ohne vorherige Destillation für die anschließende Umlagerung eingesetzt werden.

Tabelle 1 zeigt die physikalischen und spektroskopischen Daten der nach obiger allgemeiner Vorschrift hergestellten Acetylenalkohole Ia-Ie. Alle Acetylenalkohole lassen sich in Ausbeuten von 75 - 90 % herstellen.

Die angegebenen Verschiebungen sind auf Tetramethylsilan (TMS) (STMS = O ppm) bezogen.

Tabelle 1: 3,9-Dihydroxynonin und dessen an der 9-OH-Funktion geschützte Derivate

| Bsp. | R | Kp.[°C/mbar] | $^1$H-NMR ($CDCl_3$) a) δ (ppm) | $^{13}$C-NMR-($CDCl_3$) a) δ (ppm) |
|---|---|---|---|---|
| Ia | H | 108-120/0.27 | 4.35(t,1H);3.60(t,2H) 2.45(s,1H);1.20-1.75(11H); | - |
| Ib | 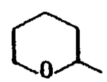 | 130-150/0.04 | 4.59(1H);4.32(t,1H); 3.86(m,1H);3.70(m,1H); 3.42-3.58(m,2H);2,47(s,1H); 1.25-1.90(m,17H) | 98.7; 85.6; 72.2; 67.5; 62.0; 61.9; 37.7; 30.8; 29.7; 29.1; 26.2; 25.6; 25.1; 19.5 |
| Ic | tert.-Butyl | 86-89/0.5 | 4.32(t,1H);3.32(t,1H); 3.05(OH);2.47(s,1H); 1.30-1.76(m,10H);1.18(s,9H) | 85.6; 72.6; 62.0; 61.6; 37.8; 30.7; 29.2; 27.7; 26.2; 25.1 |
| Id | $COCH_3$ | 106-120/0.33 | 4.37(t,1H);4.07(t,2H); 2.75(OH);2.48(s,1H); 2.07(s,3H); 1,27-1,80(m,10H) | 171.2; 85.6; 72.6; 64.6; 62.0; 37.7; 28.9; 28.6; 25.9; 25.0; 20.8 |
| Ie | $-Si(CH_3)_3$ | 87-92/0.1 | 4.28(t,1H);3.49(t,2H); 3.40(s,1H);2.35(s,1H); 1.70-1.10(10H);0.15(s,9H) | 85.9;73.0;63.1;62.3; 38.1;33.0;29.5;26.2; 25.2;-0.4 |

a) interner Standard = TMS

B) Herstellungsvorschrift zum Umlagerung des Acetylenalkohols Id in den entsprechenden α,β-ungesättigten Aldehyd IIId

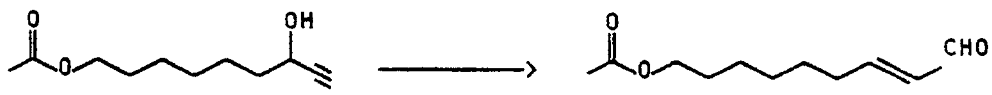

Id IIId

460 ml Vakuumpumpenöl, 15,3 g Triphenylsilanol, 8,3 g Tris(triphenylsilyloxy)vanadiumoxid, 0,82 g Benzoesäure und 91,5 g Id (0,46 mol) werden 4 Stunden bei 140 °C gerührt.

Anschließend wird das Reaktionsprodukt und nicht umgesetztes Ausgangsmaterial im Ölpumpenvakuum über eine Kolonne fraktionierend destilliert. Die Destillation ergab in 65 %iger isolierter Ausbeute NMR-spektroskopisch sauberen $\alpha,\beta$-ungesättigten Aldehyd. Das nicht umgesetzte Ausgangsmaterial läßt sich problemlos für eine erneute Umlagerung verwenden.

In analoger Weise wurden die Acetylenalkohole Ia - Ic und Ie in die entsprechenden $\alpha,\beta$-ungesättigten Aldehyde in isolierten Ausbeuten zwischen 53 - 79 % umgelagert.

C) Herstellung der Ausgangsstoffe

$C_1$) $CH_2=CH-(CH_2)_4-OH \rightarrow CH_2=CH-(CH_2)_4-O-C(CH_3)_3$

200 g Hex-1-en-6-ol werden in 250 ml Methyl-tert.-butylether gelöst und mit 100 g saurem Ionentauscher (SPC $118H^+$) versetzt. Anschließend wird bis zur Sättigung Isobutylen eingeleitet und die Reaktion per Dünnschichtchromatographie verfolgt. Nach 4 Stunden ist die Umsetzung vollständig. Fraktionierte Destillation ergibt 243 g 6-tert.-Butoxyhex-1-en mit einer GC-Reinheit von 98,5 %; $Kp_{32}$ mbar: 66 - 67 °C. Ausbeute: 75 % der Theorie.

$C_2$) Herstellung geschützter 7-Hydroxyheptanole (II) durch Hydroformylierung

1190 g 6-tert.-Butoxyhex-1-en werden in 800 g Tetrahydrofuran in Gegenwart von 1,5 g $Rh(Co)H(PO_3)_3$ und 45 g Triphenylphosphin bei 50 °C mit Synthesegas ($Co : H_2 = 1 : 1$) unter einem Druck von 100 bar für 12 Std., dann bei 60 °C/150 bar weitere 12 Std. hydroformyliert. Destillation des Reaktoraustrages liefert 121 g nicht umgesetztes Alken zurück, daneben 1009 g Aldehydgemisch mit Siedepunkt 62 - 77 °C bei 2 mbar. Als Destillationsrückstand verbleiben neben 46,5 g Katalysator etwa 70 g Hochsieder bzw. Polymere. Die Fraktionierung der 1009 g Aldehydgemisch erfolgt über eine 160 cm-Füllkörperkolonne bei 20 mbar. Erhalten wird der gewünschte unverzweigte Aldehyd (7-tert.-Butoxyheptanal) in 99,6 % Reinheit mit Siedepunkt 116 °C/20 mbar sowie der verzweigte 2-Methyl-6-tert.-Butoxyhexanal in 99,7 % Reinheit (Siedepunkt 110 - 112 °C/20 mbar).
Ausbeute: 650 g 7-tert.-Butoxyheptanal neben 280 g 2-Methyl-6-tert.-Butoxyhexanal.

In analoger Weise läßt sich z.B. der entsprechende trimethylsilylgeschützte Aldehyd herstellen.

## Ansprüche

1. 3,9-Dihydroxynonin und dessen an der 9-OH-Funktion geschützte Derivate der allgemeinen Formel I

$$HC\equiv C-\underset{OH}{\underset{|}{CH}}-(CH_2)_6-OR \qquad I,$$

in der R für Wasserstoff oder eine an sich übliche basenstabile Alkoholschutzgruppe steht.

2. 3,9-Dihydroxynonin und dessen Derivate gemäß Anspruch 1, worin R für eine tertiäre $C_4$-$C_{20}$-Alkylgruppe, eine $C_3$-$C_{20}$-Trialkylsilylgruppe, Benzyl, Benzoyl, $C_2$-$C_{10}$-Acyl, acyclische oder cyclische Acetale steht.

3. Verfahren zur Herstellung von 3,9-Dihydroxynonin und dessen Derivaten gemäß den Ansprüchen 1 und 2, dadurch gekennzeichnet, daß man einen Aldehyd der Formel II

$$O=CH-(CH_2)_6-OR \qquad II,$$

in der R eine basenstabile Alkoholschutzgruppe darstellt, mit Acetylen in einem aprotischen organischen Lösungsmittel in Gegenwart einer Base umsetzt und gegebenenfalls anschließend die Schutzgruppe abspaltet.

4. Verfahren nach Anspruch 3, dadurch gekennzeichnet, daß man als Base ein Alkylmagnesiumhalogenid oder ein Alkalimetallamid verwendet.

5. Verwendung einer Verbindung der allgemeinen Formel I gemäß Ansprüchen 1 und 2 als Zwischenprodukt zur Synthese von E-7, Z-9-Dodecadienylacetat, dem Pheromon des bekreuzten Traubenwicklers.